# EUROPEAN PATENT SPECIFICATION

(11) **EP 3 971 185 B1**
(45) Date of publication and mention of the grant of the patent: **17.04.2024**
(21) Application number: 20827289.8
(22) Date of filing: 18.06.2020
(51) Int. Cl.: C07D 407/12, C07D 209/08

(54) **METHOD FOR PREPARING INDOLE OR INDAZOLE COMPOUND**
VERFAHREN ZUR HERSTELLUNG VON INDOL- ODER INDAZOLVERBINDUNG
PROCÉDÉ DE PRÉPARATION D'UN COMPOSÉ D'INDOLE OU D'INDAZOLE

(30) Priority: 19.06.2019 KR 20190073018
(43) Date of publication of application: 23.03.2022
(73) Proprietor: LG Chem, Ltd., Seoul 07336 (KR)
(72) Inventor: LEE, Sang Dae, Daejeon 34122 (KR); PARK, Ae Ri, Daejeon 34122 (KR); CHOI, Bo Seung, Daejeon 34122 (KR); KIM, Bong Chan, Daejeon 34122 (KR)
(74) Representative: Hoffmann Eitle
(86) International application number: PCT/KR2020/007903
(87) International publication number: WO 2020/256432

(56) References cited:
- WO-A1-2009/025478
- CN-A- 106 928 121
- KR-A- 20090 075 638
- KR-A- 20130 087 283
- KR-A- 20150 022 707
- KR-A- 20170 055 499

## Description

### TECHNICAL FIELD

### Cross-reference to Related Applications

This application claims the benefit of Korean Patent Application No. 10-2019-0073018, filed on June 19, 2019, in the Korean Intellectual Property Office.

### Technical Field

The present invention relates to a novel preparation method of a pharmaceutically useful indole or indazole compound by introducing a stable intermediate.

### BACKGROUND ART

Typical diseases caused by cellular necrosis include ischemic (e.g. myocardial infarction, stroke, renal infarction), neurodegenerative, and inflammatory diseases. Cellular necrosis is an uncontrolled, accidental cell death under morbid circumstances, and studies on discovering and developing necrosis-inhibiting substances are being carried out in order to treat ischemic, neurodegenerative, and inflammatory diseases and elucidate the biological, pathological causes of cellular necrosis.

Indole derivatives have very useful structure from a medical viewpoint for the inhibition of cellular necrosis, and many results of studies on these structures have been reported. Representative examples include, for example, PCT International Publication No. WO2006/112549, which reported that the compounds have the activity for the glucokinase, PCT International Publication No. WO95/07276, which reported that the compounds are useful as anti-tumor agents and inhibitors against the production of cardiovascular system, and PCT International Publication No. WO2004/018428, which reported that the compounds can be used as antibiotics. In addition, there is PCT International Publication No. WO2009/025478 which reported a novel indole or indazole derivative structure useful for an effect of prevention or treatment and amelioration for cellular necrosis and necrosis-associated diseases.

Among these, the present invention relates to a method for preparing an indole or indazole compound, which exhibits an effect of prevention or treatment and amelioration for cellular necrosis and necrosis-associated diseases, by introducing a novel intermediate.

Conventionally, since intermediates produced during the synthesis process of indole or indazole compounds were unstable, many impurities were generated, yield is lowered, and it is difficult to be stably applied to scale up.

Thus, there is a need for developing a novel preparation method which solves limitations caused by the conventional unstable intermediate and introduces more stable intermediates.

### [PRIOR ART DOCUMENTS]

### [PATENT DOCUMENTS]

PCT International Publication WO2006/112549 (October 26, 2006)
PCT International Publication WO1995/007276 (March 16, 1995)
PCT International Publication WO2004/018428 (March 4, 2004)
PCT International Publication WO2009/025478 (February 26, 2009) WO2009/025478 and KR20090075638 disclose processes for the synthesis of indoles.

### DISCLOSURE OF THE INVENTION

### TECHNICAL PROBLEM

An aspect of the present invention provides a method for preparing a pharmaceutical useful indole or indazole compound by introducing a more stable novel intermediate in order to reduce the generation of impurities, increase yield, and stably apply to scale up.

### TECHNICAL SOLUTION

According to an aspect of the present invention that is defined in detail in the claims, there is provided a method for preparing a compound represented by Formula 2 below by comprising a compound represented by Formula 1 below as a reaction intermediate:

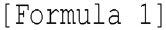

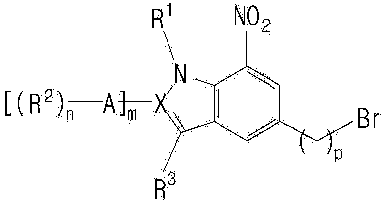

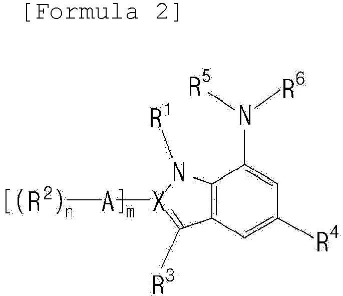

In Formulae above,
n is an integer of 1 to 3,
m is 0 or 1,
p is an integer of 1 to 3,
A represents phenyl, or represents 5-membered heteroaryl or heterocycle which each contains 1 to 3 heteroatoms selected from among N, O and S atoms and is optionally substituted by R, wherein R represents hydrogen or represents C₁-C₄-alkyl optionally substituted by hydroxy or amino,
X represents C or N, with the proviso that m is 0 when X is N and m is 1 when X is C,
R¹ represents hydrogen, C₁-C₆-alkyl or -(CH₂)ᵣNR⁸R⁹, wherein r is an integer of 2 to 5, R⁸ and R⁹ each independently represent hydrogen or C₁-C₃-alkyl, with the proviso that R¹ is hydrogen when X is N,
R² represents hydrogen, halogen or C₁-C₆-alkoxy, or represents -(CH₂)ₛCO₂R⁸, -(CH₂)ₛOR⁸, -(CH₂)ₛNR⁸R⁹, -NHR¹⁰, - N(H)S(O)₂R⁸, or -NHC(O)R¹⁰, or represents -(CH₂)ₛ-heterocycle-R¹⁰ in which the heterocycle moiety is a 5-6 membered ring containing one or two heteroatoms selected from among N, O, and S atoms, wherein s is an integer of 0 to 3, R⁸ and R⁹ are as defined above, and R¹⁰ represents hydrogen, oxo, C₁-C₆-alkylcarbonyl, C₁-C₆-alkoxy or C₁-C₆- alkyl, or represents a 5-6 membered heterocycle containing one or two nitrogen atoms as a heteroatom,
R³ represents hydrogen, halogen, C₁-C₆-alkyl or phenyl, or represents -(CH₂)_{g}-heterocycle in which the heterocycle is a 5-6 membered ring containing one or two heteroatoms selected from among N and O atoms, wherein g is an integer of 1 to 3, with the proviso that R³ is hydrogen or phenyl when X is N,
R⁴ represents -YR¹¹, wherein Y is a direct bond or represents -(CR⁸R⁹)ₑY'-, wherein e is an integer of 0 to 3, and R⁸ and R⁹ are the same as defined above,
Y' is selected from the group consisting of -O-, - C(O)-, and -C(O)O-, R¹¹ is selected from the group consisting of hydrogen, halogen, C₁-C₆-alkyl, and - (CH₂)ₜB-R¹³, t is an integer of 0 to 3, B represents a 5-6 membered heterocycle containing one or two heteroatoms selected from among N, O, and S atoms, or represents C₆-C₁₀-aryl, R¹³ represents hydrogen, cyano, halogen, hydroxy, oxo, thiol, carboxy or carboxy-C₁-C₆-alkyl, with the proviso that R⁴ is hydrogen or C₁-C₆-alkyl when X is N,
R⁵ represents hydrogen, C₁-C₆-alkyl, C₃-C₆-cycloalkyl, heterocycle or heterocyclyl-C₁-C₆-alkyl, wherein the heterocycle is a 3-8 membered ring containing one to three heteroatoms selected from among N and O atoms, with the proviso that R⁵ is hydrogen when X is N,
R⁶ represents -(CR⁸R⁹)ᵤ-Z-D-W-R¹⁴, wherein u is an integer of 0 to 3, Z represents a direct bond or is selected from the group consisting of -C(O)- and -C(0)0-, D represents a direct bond, C₄-C₆-cycloalkyl, 5-6 membered heteroaryl containing one or two N atoms, or a 5-6 membered heterocycle containing one or two heteroatoms selected from among N, O and S atoms, W represents a direct bond, or -NR⁸-,-C(O)-, - C(O)O-, -C(O)NR¹²- or -S(O)_{y}-, R¹² represents hydrogen, C₁-C₃-alkyl or C₆-C₁₀-aryl, y is an integer of 1 or 2, and R¹⁴ represents hydrogen, hydroxy, C₁-C₆-alkyl, a 5-6 membered heterocycle containing one to three heteroatoms selected from among N, O and S atoms, or C₆-C₁₀-Ar-C₁-C₆-alkyl, with the proviso that when X is N, R⁶ represents C₄-C₆-cycloalkyl, or represents a 5-6 membered heterocycle containing one or two heteroatoms selected from among N, O, and S atoms, and
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo.

### ADVANTAGEOUS EFFECTS

According to the present invention, it is possible to solve the limitations caused by conventional unstable intermediates to reduce the generation of impurities, prepare an indole or indazole compound in excellent yield, and be stably applied to scale up.

### MODE FOR CARRYING OUT THE INVENTION

Hereinafter, the present invention will be described in more detail. Here, terms or words used in the specification and claims should not be interpreted as being limited to a conventional or dictionary meaning, and should be interpreted as the meaning and concept that accord with the technical spirit on the grounds of the principle that the inventor can appropriately define the concept of the term in order to explain the invention in the best way.

In the definition for the substituents of Formulae according to the present invention, the term "alkyl" refers to an aliphatic hydrocarbon radical. Alkyl may be "saturated alkyl" which does not include an alkenyl or alkynyl moiety, or "unsaturated alkyl" which includes at least one alkenyl or alkynyl moiety. The term "alkenyl" refers to a group containing at least one carbon-carbon double bond, and the term "alkynyl" refers to a group containing at least one carbon-carbon triple bond Alkyl may be branched or linear when used alone or in a composite form such as alkoxy.

The alkyl group may have 1 to 20 carbon atoms unless otherwise defined. The alkyl group may be medium-sized alkyl having 1 to 10 carbon atoms. The alkyl group may be lower alkyl having 1 to 6 carbon atoms. A typical alkyl group includes, but is not limited to, methyl, ethyl, propyl, isopropyl, butyl, isobutyl, t-butyl, pentyl, hexyl, ethenyl, propenyl, butenyl, etc. For example, C₁-C₄-alkyl has 1 to 4 carbon atoms in the alkyl chain, and is selected from the group consisting of methyl, ethyl, propyl, iso-propyl, n-butyl, iso-butyl, sec-butyl and t-butyl.

The term "alkoxy" refers to alkyloxy having 1 to 10 carbon atoms unless otherwise defined.

The term "cycloalkyl" refers to a saturated aliphatic 3-10 membered ring unless otherwise defined. A typical cycloalkyl group includes, but is not limited to, cyclopropyl, cyclobutyl, cyclopentyl, cyclohexyl, etc.

The term "aryl" includes at least one ring having a covalent π electron system, for example, a monocyclic or fused polycyclic (i.e., rings that share adjacent pairs of carbon atoms) group. That is, unless otherwise defined herein, aryl refers to an aromatic 4-10 membered, preferably 6-10 membered, monocyclic or multicyclic ring including phenyl, naphthyl, etc.

The term "heteroaryl" refers to an aromatic 3-10 membered ring, preferably 4-8 membered ring, more preferably 5-6 membered ring, which contains 1 to 3 heteroatoms selected from the group consisting of N, O and S and may be fused with benzo or C₃-C₈ cycloalkyl unless otherwise defined. Examples of monocyclic heteroaryl include, but are not limited to, thiazole, oxazole, thiophene, furan, pyrrole, imidazole, isoxazole, isothiazole, pyrazole, triazole, triazine, thiadiazole, tetrazole, oxadiazole, pyridine, pyridazine, pyrimidine, pyrazine and the like. Examples of bicyclic heteroaryl include, but are not limited to, indole, indoline, benzothiophene, benzofuran, benzimidazole, benzoxazole, benzisoxazole, benzthiazole, benzthiadiazole, benztriazole, quinoline, isoquinoline, purine, furopyridine and the like.

The term "heterocycle" refers to a 3-10 membered ring, preferably 4-8 membered ring, more preferably 5-6 membered ring, which contains 1 to 3 heteroatoms selected from the group consisting of N, O and S and may be fused with benzo or C₃-C₈ cycloalkyl, and is saturated or contains 1 or 2 double bonds, unless otherwise defined. Examples of the heterocycle include, but are not limited to, pyrroline, pyrrolidine, imidazoline, imidazolidine, pyrazoline, pyrazolidine, pyran, piperidine, morpholine, thiomorpholine, piperazine, hydrofuran, and the like.

Unless otherwise defined, terms and abbreviations used herein may be interpreted as meanings commonly understood by those skilled in the art to which the present invention pertains.

The present invention relates to a method for preparing a pharmaceutically useful indole or indazole compound, the method preparing a compound represented by Formula 2 below by comprising a compound represented by Formula 1 below as a reaction intermediate: 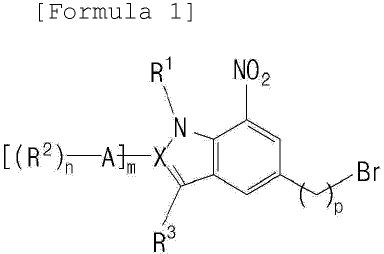
wherein, in Formula 1 above,
n is an integer of 1 to 3,
m is 0 or 1,
p is an integer of 1 to 3,
A represents phenyl, or represents 5-membered heteroaryl or heterocycle which each contains 1 to 3 heteroatoms selected from among N, O and S atoms and is optionally substituted by R, wherein R represents hydrogen or represents C₁-C₄-alkyl optionally substituted by hydroxy or amino,
X represents C or N, with the proviso that m is 0 when X is N and m is 1 when X is C,
R¹ represents hydrogen, C₁-C₆-alkyl or -(CH₂)ᵣNR⁸R⁹, wherein r is an integer of 2 to 5, R⁸ and R⁹ each independently represent hydrogen or C₁-C₃-alkyl, with the proviso that R¹ is hydrogen when X is N,
R² represents hydrogen, halogen or C₁-C₆-alkoxy, or represents - (CH₂)ₛCO₂R⁸, -(CH₂)ₛOR⁸, - (CH₂)ₛNR⁸R⁹, -NHR¹⁰,-N(H)S(O)₂R⁸, or -NHC(O)R¹⁰, or represents -(CH₂)ₛ-heterocycle-R¹⁰ in which the heterocycle moiety is a 5-6 membered ring containing one or two heteroatoms selected from among N, O, and S atoms, wherein s is an integer of 0 to 3, R⁸ and R⁹ are as defined above, and R¹⁰ represents hydrogen, oxo, C₁-C₆-alkylcarbonyl, C₁-C₆-alkoxy or C₁-C₆- alkyl, or represents a 5-6 membered heterocycle containing one or two nitrogen atoms as a heteroatom, and
R³ represents hydrogen, halogen, C₁-C₆-alkyl or phenyl, or represents -(CH₂)_{g}-heterocycle in which the heterocycle is a 5-6 membered ring containing one or two heteroatoms selected from among N and O atoms, wherein g is an integer of 1 to 3, with the proviso that R³ is hydrogen or phenyl when X is N.

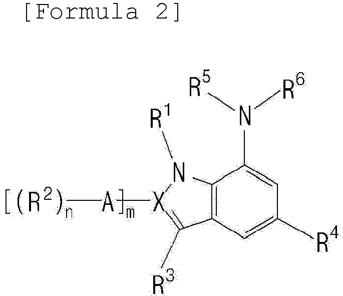

In Formula 2 above, n, m, X, A, R¹, R², and R³ are the same as defined above,
R⁴ represents -YR¹¹, wherein Y is a direct bond or represents -(CR⁸R⁹)ₑY'-, wherein e is an integer of 0 to 3, and R⁸ and R⁹ are the same as defined above,
Y' is selected from the group consisting of -O-, - C(O)-, and -C(O)O-, R¹¹ is selected from the group consisting of hydrogen, halogen, C₁-C₆-alkyl, and -(CH₂)ₜB-R¹³, t is an integer of 0 to 3, B represents a 5-6 membered heterocycle containing one or two heteroatoms selected from among N, O, and S atoms, or represents C₆-C₁₀-aryl, R¹³ represents hydrogen, cyano, halogen, hydroxy, oxo, thiol, carboxy or carboxy-C₁-C₆-alkyl, with the proviso that R⁴ is hydrogen or C₁-C₆-alkyl when X is N,
R⁵ represents hydrogen, C₁-C₆-alkyl, C₃-C₆-cycloalkyl, heterocycle or heterocyclyl-C₁-C₆-alkyl, wherein the heterocycle is a 3-8 membered ring containing one to three heteroatoms selected from among N and O atoms, with the proviso that R⁵ is hydrogen when X is N,
R⁶ represents -(CR⁸R⁹)ᵤ-Z-D-W-R¹⁴, wherein u is an integer of 0 to 3, Z represents a direct bond or is selected from the group consisting of -C(O)- and -C(O)O-, D represents a direct bond, C₄-C₆-cycloalkyl, 5-6 membered heteroaryl containing one or two N atoms, or a 5-6 membered heterocycle containing one or two heteroatoms selected from among N, O and S atoms, W represents a direct bond, or -NR⁸-, -C(O)-, - C(O)O-, -C(O)NR¹²- or -S(O)_{y}-, R¹² represents hydrogen, C₁-C₃-alkyl or C₆-C₁₀-aryl, y is an integer of 1 or 2, and R¹⁴ represents hydrogen, hydroxy, C₁-C₆-alkyl, a 5-6 membered heterocycle containing one to three heteroatoms selected from among N, O and S atoms, or C₆-C₁₀-Ar-C₁-C₆-alkyl, with the proviso that when X is N, R⁶ represents C₄-C₆-cycloalkyl, or a 5-6 membered heterocycle containing one or two heteroatoms selected from among N, O, and S atoms, and
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo.

Specifically, in Formula 1 and Formula 2 above, R¹ above may be hydrogen, C₁-C₆-alkyl, or di(C₁-C₃-alkyl)amino-C₂-C₃-alkyl.

In addition, R² above may represent hydrogen, halogen, carboxy, carboxy-C₁-C₃-alkyl, C₁-C₃-alkoxycarbonyl, C₁-C₃-alkoxycarbonyl-C₁-C₃-alkyl, hydroxy-C₁-C₃-alkyl optionally substituted by one oxo group, C₁-C₃-alkoxy, -(CH₂)ₛNR⁸R⁹, - NHR¹⁰, -N(H)S(O)₂R¹⁰ or -NHC(O)₂R¹⁰ or may be -(CH₂)ₛ-heterocycle-R¹⁰, wherein heterocycle, s, R⁸, R⁹ and R¹⁰ are as defined above.

Further, R³ may represent hydrogen, methyl or halogen, phenyl optionally substituted by C₁-C₃-alkoxy, or may be heterocyclyl-C₁-C₃-alkylene in which the heterocycle is a 5-6 membered ring containing one or two heteroatoms selected from N and O atoms and optionally substituted with one or two oxo groups.

In addition, Y above may be selected from the group consisting of a direct bond, -O-, -C(O)-, and -CH₂C(O)-.

In addition, R¹¹ above may be selected from the group consisting of hydrogen, methyl, ethyl, phenyl, fluoro, chloro, 2-carboxy-pyrrolidin-1-yl, pyrrolidin-1-yl, 4-acetic acid-1,3-thiazolin-2-yl, -CH₂-(1,1-dioxo-thiomorpholin-4-yl) and - CH₂-(2-oxopiperazin-4-yl).

Also, in Formula 2 above, R⁵ above may be hydrogen, methyl, or isobutyl.

R⁶ above may be selected from the group consisting of cyclobutyl, cyclopentyl, cyclohexyl, 4-methyl-cyclopentyl, 4,4-difluorocyclohexyl, or D may be selected from the group consisting of cyclopentyl, cyclohexyl, pyrrolidine, tetrahydropyran, tetrahydrofuran and piperidine, and W may represent a direct bond or may represent -SO₂-, -CO-, -C(O)O-or -CONR¹²-, wherein R¹² is the same as defined above.

As an embodiment of the present invention, the compound represented by Formula 1 above may be 5-(bromomethyl)-7-nitro-2-phenyl-1H-indole, and the compound represented by Formula 2 above may be 4-((2-phenyl-7-((tetrahydro-2H-pyran-4-yl)amino)-1H-indol-5-yl)methyl)thiomorpholine 1,1-dioxide.

The compound represented by Formula 1 above is prepared by brominating the compound represented by Formula 3 below:

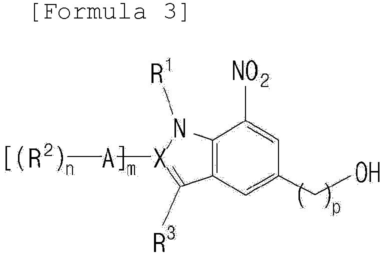

In Formula 3 above, n, m, p, X, A, R¹, R², and R³ are the same as defined above,

The brominating step is not specifically limited, and may be performed by a conventional manner.

The compound represented by Formula 3 above is brominated to produce a more stable intermediate represented by Formula 1 above, thereby reducing the generation of impurities, improving reaction yield, and being stably applied to scale up.

The compound represented by Formula 1 above may be prepared by reacting the compound represented by Formula 3 above in the presence of at least one compound selected from the group consisting of PBr₃, SOBr₂, HBr and LiBr, and more preferably Pbr₃.

The preparation method of the present invention maycomprise, after forming the intermediate of Formula 1 above, the steps for: substituting the compound represented by Formula 1 above to prepare a compound represented by Formula 4 below; reducing the compound represented by Formula 4 to prepare a compound represented by Formula 5; and reacting the compound represented by Formula 5 with ketone or aldehyde to prepare a compound represented by Formula 2 above.

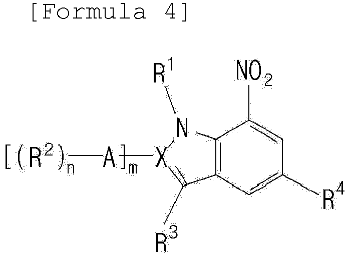

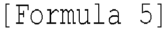

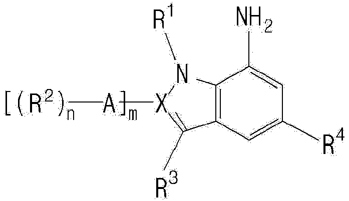

In Formula 4 and Formula 5 above, n, m, X, A, R¹, R², R³, and R⁴ are the same as defined above,

Hereinafter, embodiments of the present invention will be described in detail so that a person with ordinary skill in the art can easily practice the present invention. The present invention may, however, be embodied in different forms and should not be construed as limited to the embodiments set forth herein.

### Example

### Step a) Synthesis of 4-((7-nitro-2-phenyl-1H-indol-5-yl)methyl)thiomorpholine 1,1-dioxide

5-(hydroxymethyl)-7-nitro-2-phenyl-1H-indole (4.6 kg) and THF (46 L) were added to a reactor and stirred at room temperature, the reactor was cooled to a temperature of 0 °C, and then PBr₃ (2.8 kg) was added while the internal temperature of the reaction was maintained below 20 °C. After the completion of the addition, the temperature of the reactor was raised to 25 °C, the mixture was stirred for 1 hour, and the reaction was terminated when the reaction mixture was analyzed by sampling to show 1.0% or less of the peak of 5-(hydroxymethyl)-7-nitro-2-phenyl-1H-indole.

The completion of the reaction was confirmed, the reaction mixture was cooled to 0 °C, and 1,1-dioxothiomorpholine hydrochloride (4.4 kg) and N,N-diisopropylethylamine (DIPEA) (8.0 kg) were added while the internal temperature of the reaction was maintained below 20 °C. After the completion of the addition, the temperature of the reactor was raised to 50 °C, the mixture was stirred for 4 hours, and the reaction was terminated when the reaction mixture was analyzed by sampling to show 1.0% or less of the peak of 5-(bromomethyl)-7-nitro-2-phenyl-1H-indole.

After confirming the completion of the reaction, the reactor was cooled to a temperature of 0 °C, water (46.0 L) was added thereto, the reaction mixture was stirred, and the organic solvent was distilled under reduced pressure. After the distillation under reduced pressure, ethyl acetate (EtOAc) (48.0 L) was additionally added thereto, and the organic solvent was distilled under reduced pressure again to obtain 4-((7-nitro-2-phenyl-1H-indol-5-yl)methyl)thiomorpholine 1,1-dioxide as a solid, and then filtered. The resultant was washed with water (26.0 L) and ethanol (26.0 L) and dried under N₂ pressure for 16 hours to synthesize crude 4-((7-nitro-2-phenyl-1H-indol-5-yl)methyl)thiomorpholine 1,1-dioxide (6.4 kg, purity: 92.9%).

For purification, crude 4-((7-nitro-2-phenyl-1H-indol-5-yl)methyl)thiomorpholine 1,1-dioxide above was added to a mixture of DMF (6.0 L) and toluene (60.0 L), stirred, heated to 120 °C, stirred for 2 hours, and then stirred for another 2 hours at room temperature again. Toluene (60.0 L) was additionally added thereto and stirred for 2 another hours, and then a solid in the solution was filtered and washed with toluene (20.0 L) to synthesize 4-((7-nitro-2-phenyl-1H-indol-5-yl)methyl)thiomorpholine 1,1-dioxide (5.1 kg, yield: 77.0% overall, purity: 98.4%).

¹H NMR (500MHz, DMSO-d₆) δ 11.63 (s, 1H), 8.07 (s, 1H), 8.04 (m, 3H), 7.49 (t, J= 7.7 Hz, 2H) 7.38 (d, J= 7.3 Hz, 1H), 7.14 (s, 1H), 3.79 (s, 2H), 3.12 (m, 4H), 2.84 (m, 4H).

### Step b) Synthesis of 4-((7-amino-2-phenyl-1H-indol-5-yl)methyl)thiomorpholine 1,1-dioxide

4-((7-nitro-2-phenyl-1H-indol-5-yl)methyl)thiomorpholine 1,1-dioxide (0.60 kg), THF/methanol/H₂O (1:1:1, 3.0 L), and NH₄Cl (124 g) were added to a reactor at room temperature and stirred, and then Fe (480 g) was added thereto. The reactor was heated to a temperature of 60 °C, the reaction mixture was stirred for 1 hour or more, and then the reaction was terminated when the reaction mixture was analyzed to show 0.5% or less of the peak of 4-((7-nitro-2-phenyl-1H-indol-5-yl)methyl)thiomorpholine 1,1-dioxide.

The completion of the reaction was confirmed, THF (3.0 L) was added to the reaction mixture and stirred for 10 minutes, and then filtered with celite, and the resultant was washed with THF/H₂O (1:1, 3.0 L). The filtrate was under reduced pressure and THF and methanol were distilled to form a solid, and the reaction mixture was stirred at room temperature for 2 hours or more. The resultant was filtered and washed with the washing solution [1st H₂O: 500 mL, 2nd ethanol/H₂O = 1/3, 1.0 L], and then dried under N₂ pressure for 16 hours to synthesize 4-((7-amino-2-phenyl-1H-indol-5-yl)methyl)thiomorpholine 1,1-dioxide [0.45 kg (yield: 81.3%, purity: 97.2%)].

¹H NMR (500MHz, DMSO-d₆) δ 10.89 (s, 1H), 7.80 (m, 2H), 7.48 (t, J= 7.7 Hz, 2H) 7.30 (d, J= 7.3 Hz, 1H), 6.74 (m, 2H), 6.34 (s, 1H), 5.15 (s, 2H), 3.07 (m, 4H), 2.87 (m, 4H).

### Step c) Synthesis of 4-((2-phenyl-7-((tetrahydro-2H-pyran-4-yl)amino)-1H-indol-5-yl)methyl)thiomorpholine 1,1-dioxide

4-((7-amino-2-phenyl-1H-indol-5-yl)methyl)thiomorpholine 1,1-dioxide (3.8 kg), tetrahydro-4H-pyran-4-one (1.3 kg), acetic acid (AcOH) (4.6 kg), and isopropyl acetate (23.2 L) were added to a reactor and stirred, and then NaBH(OAc)₃ (4.6 kg) was added thereto, followed by stirring at room temperature for 1 hour or more, and the reaction was terminated when the reaction mixture was analyzed to show 0.5% or less of the peak of 4-((7-amino-2-phenyl-1H-indol-5-yl)methyl)thiomorpholine 1,1-dioxide

The completion of the reaction was confirmed, a 1N NaOH aqueous solution (23.2 L) was added to the reaction mixture, followed by stirring for another 1 hour or more, and then the organic solvent was distilled under reduced pressure. The solid present in the remaining aqueous solution was filtered, washed with water (38.7 L) and t-butyl methyl ether (38.7 L), and dried under N₂ pressure for 16 hours to synthesize 4-((2-phenyl-7-((tetrahydro-2H-pyran-4-yl)amino)-1H-indol-5-yl)methyl)thiomorpholine 1,1-dioxide (3.9 kg, yield: 82.4%, purity: 98.5%).

¹H NMR (500MHz, DMSO-d₆) δ 10.96 (s, 1H), 7.80 (m, 2H), 7.48 (t, J= 7.7 Hz, 2H) 7.30 (d, J= 7.3 Hz, 1H), 6.74 (s, 2H), 6.30 (s, 1H), 5.36 (m, 1H), 3.95 (m, 2H), 3.62 (s, 2H), 3.50 (m, 2H), 3.09 (m, 4H), 2.87 (m, 4H), 2.05 (m, 2H), 1.45 (m, 2H).

### Comparative Example

### Step a) Synthesis of 4-((7-nitro-2-phenyl-1H-indol-5-yl)methyl)thiomorpholine 1,1-dioxide

5-(hydroxymethyl)-7-nitro-2-phenyl-1H-indole (804 mg, 3 mmol) was dissolved in THF (10 mL) at room temperature, and imidazole (408 mg, 6 mmol) and triphenylphosphine (1.52 g, 6 mmol) were added thereto. Iodine (453 mg, 3.9 mmol) was added thereto and stirred for 2 hours. When the reaction was terminated, the reaction mixture was filtered with celite and used in the following reaction without additional purification.

The obtained 5-iodomethyl-2-phenyl-7-nitro-1H-indole (850 mg, 2.5 mmol) was dissolved in THF (10 mL), and 1, 1-dioxo-thiomorpholine (405 mg, 2.25 mmol) was added and stirred for 2 hours. When the reaction was completed, the resultant was diluted with water, organic matter was extracted with ethyl acetate (EtOAc), and then dried with anhydrous magnesium sulfate and filtered. The residue was recrystallized with DCM and hexane to obtain 4-((7-nitro-2-phenyl-1H-indol-5-yl)methyl)thiomorpholine 1,1-dioxide (625 mg, yield: 65%, purity: 97.2%).

Next, 4-((2-phenyl-7-((tetrahydro-2H-pyran-4-yl)amino)-1H-indol-5-yl)methyl)thiomorpholine 1,1-dioxide was prepared in the same manner as in Step b) and Step c) of Example 1.

Referring to Example and Comparative Example, it may be confirmed that the yield of Step a) of Example obtained by synthesizing 4-((7-nitro-2-phenyl-1H-indol-5-yl)methyl)thiomorpholine 1,1-dioxide from 5-(hydroxymethyl)-7-nitro-2-phenyl-1H-indole via 5-(bromomethyl)-7-nitro-2-phenyl-1H-indole intermediate according to the present invention was 77.0%, which was remarkably improved compared to the yield of Step a) of Comparative Example which was 65%.

## Claims

1. A method for preparing a compound represented by Formula 2 below comprising a compound represented by Formula 1 below as a reaction intermediate, wherein the compound represented by Formula 1 below is prepared by brominating the compound represented by Formula 3 below: 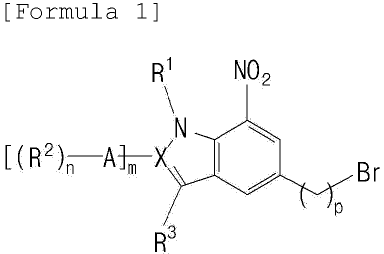 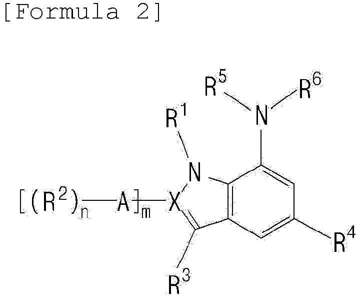 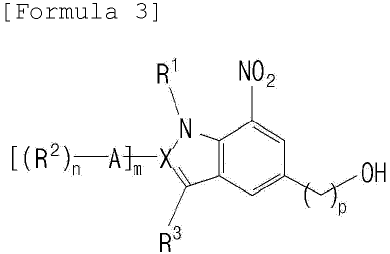
wherein, in Formulae above,
n is an integer of 1 to 3,
m is 0 or 1,
p is an integer of 1 to 3,
A represents phenyl, or represents 5-membered heteroaryl or heterocycle which each contains 1 to 3 heteroatoms selected from among N, O and S atoms and is optionally substituted by R, wherein R represents hydrogen or represents C₁-C₄-alkyl optionally substituted by hydroxy or amino,
X represents C or N, with the proviso that m is 0 when X is N and m is 1 when X is C,
R¹ represents hydrogen, C₁-C₆-alkyl or -(CH₂)ᵣNR⁸R⁹, wherein r is an integer of 2 to 5, R⁸ and R⁹ each independently represent hydrogen or C₁-C₃-alkyl, with the proviso that R¹ is hydrogen when X is N,
R² represents hydrogen, halogen or C₁-C₆-alkoxy, or represents - (CH₂)ₛCO₂R⁸, -(CH₂)ₛOR⁸, - (CH₂)ₛNR⁸R⁹, -NHR¹⁰,-N(H)S(O)₂R⁸, or -NHC(O)R¹⁰, or represents -(CH₂)ₛ-heterocycle-R¹⁰ in which the heterocycle moiety is a 5-6 membered ring containing one or two heteroatoms selected from among N, O, and S atoms, wherein s is an integer of 0 to 3, R⁸ and R⁹ are as defined above, and R¹⁰ represents hydrogen, oxo, C₁-C₆-alkylcarbonyl, C₁-C₆-alkoxy or C₁-C₆- alkyl, or represents a 5-6 membered heterocycle containing one or two nitrogen atoms as a heteroatom,
R³ represents hydrogen, halogen, C₁-C₆-alkyl or phenyl, or represents -(CH₂)_{g}-heterocycle in which the heterocycle is a 5-6 membered ring containing one or two heteroatoms selected from among N and O atoms, wherein g is an integer of 1 to 3, with the proviso that R³ is hydrogen or phenyl when X is N,
R⁴ represents -YR¹¹, wherein Y is a direct bond or represents -(CR⁸R⁹)ₑY'-, wherein e is an integer of 0 to 3, and R⁸ and R⁹ are the same as defined above,
Y' is selected from the group consisting of -O-, -C(O)-, and -C(O)O-, R11 is selected from the group consisting of hydrogen, halogen, C₁-C₆-alkyl, and -(CH₂)ₜB-R¹³, t is an integer of 0 to 3, B represents a 5-6 membered heterocycle containing one or two heteroatoms selected from among N, O, and S atoms, or represents C₆-C₁₀-aryl, R¹³ represents hydrogen, cyano, halogen, hydroxy, oxo, thiol, carboxy or carboxy-C₁-C₆-alkyl, with the proviso that R⁴ is hydrogen or C₁-C₆-alkyl when X is N,
R⁵ represents hydrogen, C₁-C₆-alkyl, C₃-C₆-cycloalkyl, heterocycle or heterocyclyl-C₁-C₆-alkyl, wherein the heterocycle is a 3-8 membered ring containing one to three heteroatoms selected from among N and O atoms, with the proviso that R⁵ is hydrogen when X is N, and
R⁶ represents -(CR⁸R⁹)ᵤ-Z-D-W-R¹⁴, wherein u is an integer of 0 to 3, Z represents a direct bond or is selected from the group consisting of -C(O)- and -C(O)O-, D represents a direct bond, C₄-C₆-cycloalkyl, or 5-6 membered heteroaryl containing one or two N atoms, or represents a 5-6 membered heterocycle containing one or two heteroatoms selected from among N, O and S atoms, W represents a direct bond, or -NR⁸-,-C(O)-, -C(O)O-, -C(O)NR¹²- or -S(O)_{y}-, R¹² represents hydrogen, C₁-C₃-alkyl or C₆-C₁₀-aryl, y is an integer of 1 or 2, and R¹⁴ represents hydrogen, hydroxy, C₁-C₆-alkyl, a 5-6 membered heterocycle containing one to three heteroatoms selected from among N, O and S atoms, or C₆-C₁₀-Ar-C₁-C₆-alkyl, with the proviso that when X is N, R⁶ represents C₄-C₆-cycloalkyl, or represents a 5-6 membered heterocycle containing one or two heteroatoms selected from among N, O, and S atoms,
wherein alkyl, alkoxy, aryl, cycloalkyl, heterocycle and heteroaryl may be optionally substituted, and the substituents are one or more selected from the group consisting of hydroxy, C₁-C₆-alkylamino, di(C₁-C₆-alkyl)amino, carboxy, C₁-C₆-alkyl, C₁-C₆-alkoxy, carboxy-C₁-C₆-alkyl and oxo.

2. The method of claim 1, wherein the compound represented by Formula 1 above is prepared by reacting the compound represented by Formula 3 above in the presence of at least one compound selected from the group consisting of PBr₃, SOBr₂, HBr and LiBr.

3. The method of claim 1, comprising the steps for:
substituting the compound represented by Formula 1 above to prepare a compound represented by Formula 4 below;
reducing the compound represented by Formula 4 above to prepare a compound represented by Formula 5; and
reacting the compound represented by Formula 5 with ketone or aldehyde to prepare a compound represented by Formula 2 above: 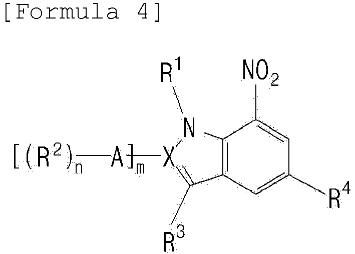 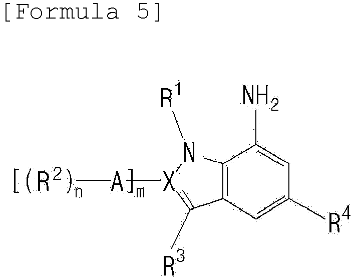 wherein, in Formulae above, n, m, X, A, R1, R2, R3, and R4 are the same as defined in claim 1.

## Patentansprüche

1. Verfahren zur Herstellung einer Verbindung, dargestellt durch die nachstehende Formel 2, umfassend eine durch die nachstehende Formel 1 dargestellte Verbindung als Reaktionsintermediat, wobei die durch die nachstehende Formel 1 dargestellte Verbindung durch Bromieren der durch die nachstehende Formel 3 dargestellten Verbindung hergestellt wird: 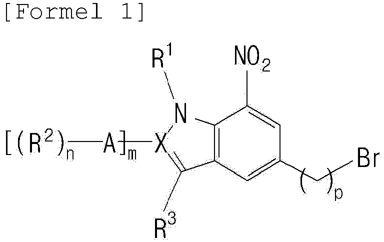 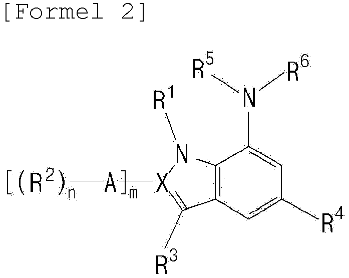 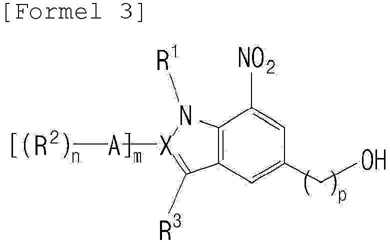
wobei in den obigen Formeln
n eine ganze Zahl von 1 bis 3 ist,
m 0 oder 1 ist,
p eine ganze Zahl von 1 bis 3 ist,
A für Phenyl steht oder für ein 5-gliedriges Heteroaryl oder einen 5-gliedrigen Heterocyclus, jeweils enthaltend 1 bis 3 Heteroatome, ausgewählt aus N-, O- und S-Atomen, steht und optional mit R substituiert ist, wobei R für Wasserstoff steht oder für C₁-C₄-Alkyl, das optional mit Hydroxy oder Amino substituiert ist, steht,
X für C oder N steht, mit der Maßgabe, dass m 0 ist, wenn X N ist, und m 1 ist, wenn X C ist,
R¹ für Wasserstoff, C₁-C₆-Alkyl oder -(CH₂)ᵣNR⁸R⁹ steht, worin r eine ganze Zahl von 2 bis 5 ist, R⁸ und R⁹ jeweils unabhängig voneinander für Wasserstoff oder C₁-C₃-Alkyl stehen, mit der Maßgabe, dass R¹ Wasserstoff ist, wenn X N ist,
R² für Wasserstoff, Halogen oder C₁-C₆-Alkoxy steht, oder für -(CH₂)ₛCO₂R⁸, -(CH₂)ₛOR⁸, -(CH₂)ₛNR⁸R⁹, -NHR¹⁰, -N(H)S(O)₂R⁸ oder -NHC(O)R¹⁰ steht oder für -(CH₂)ₛ-Heterocyclus-R¹⁰ steht, worin die Heterocyclus-Gruppe ein 5-6-gliedriger Ring ist, der ein oder zwei Heteroatome enthält, die aus N, O- und S-Atomen ausgewählt sind, wobei s eine ganze Zahl von 0 bis 3 ist, R⁸ und R⁹ wie oben definiert sind, und R¹⁰ für Wasserstoff, Oxo, C₁-C₆-Alkylcarbonyl, C₁-C₆-Alkoxy oder C₁-C₆-Alkyl steht oder für einen 5-6-gliedrigen Heterocyclus steht, der ein oder zwei Stickstoffatome als Heteroatom enthält,
R³ für Wasserstoff, Halogen, C₁-C₆-Alkyl oder Phenyl steht oder für -(CH₂)_{g}-Heterocyclus steht, worin der Heterocyclus ein 5-6-gliedriger Ring ist, der ein oder zwei Heteroatome, ausgewählt aus N- und O-Atomen, enthält, wobei g eine ganze Zahl von 1 bis 3 ist, mit der Maßgabe, dass R³ Wasserstoff oder Phenyl ist, wenn X N ist,
R⁴ für -YR¹¹ steht, wobei Y eine direkte Bindung ist oder für -(CR⁸R⁹)ₑY'- steht, wobei e eine ganze Zahl von 0 bis 3 ist, und R⁸ und R⁹ die gleichen, wie oben definiert, sind,
Y' aus der Gruppe, bestehend aus -O-, -C(O)- und -C(O)O-, ausgewählt ist, R¹¹ aus der Gruppe, bestehend aus Wasserstoff, Halogen, C₁-C₆-Alkyl und -(CH₂)ₜB-R¹³, ausgewählt ist, t eine ganze Zahl von 0 bis 3 ist, B für einen 5-6-gliedrigen Heterocyclus steht, der ein oder zwei Heteroatome, ausgewählt aus N, O- und S-Atomen, enthält, oder für C₆-C₁₀-Aryl steht, R¹³ für Wasserstoff, Cyano, Halogen, Hydroxy, Oxo, Thiol, Carboxy oder Carboxy-C₁-C₆-alkyl steht, mit der Maßgabe, dass R⁴ Wasserstoff oder C₁-C₆-Alkyl ist, wenn X N ist,
R⁵ für Wasserstoff, C₁-C₆-Alkyl, C₃-C₆-Cycloalkyl, Heterocyclus oder Heterocyclyl-C₁-C₆-alkyl steht, wobei der Heterocyclus ein 3-8-gliedriger Ring ist, der ein bis drei Heteroatome, ausgewählt aus N- und O-Atomen, enthält, mit der Maßgabe, dass R⁵ Wasserstoff ist, wenn X N ist, und
R⁶ für -(CR⁸R⁹)ᵤ-Z-D-W-R¹⁴ steht, worin u eine ganze Zahl von 0 bis 3 ist, Z für eine direkte Bindung steht oder aus der Gruppe, bestehend aus -C(O)- und -C(O)O-, ausgewählt ist, D für eine direkte Bindung, C₄-C₆-Cycloalkyl oder 5-6-gliedriges Heteroaryl, enthaltend ein oder zwei N-Atome, steht oder für einen 5-6-gliedrigen Heterocyclus, der ein oder zwei Heteroatome, ausgewählt aus N-, O- und S-Atomen, enthält, steht, W für eine direkte Bindung oder -NR⁸-, -C(O)-, -C(O)O-, -C(O)NR¹²- oder -S(O)_{y}- steht, R¹² für Wasserstoff, C₁-C₃-Alkyl oder C₆-C₁₀-Aryl steht, y eine ganze Zahl von 1 oder 2 ist, und R¹⁴ für Wasserstoff, Hydroxy, C₁-C₆-Alkyl, einen 5-6-gliedrigen Heterocyclus, der ein bis drei Heteroatome, ausgewählt aus N-, O- und S-Atomen, enthält, oder C₆-C₁₀-Ar-C₁-C₆-Alkyl steht, mit der Maßgabe, dass R⁶ für C₄-C₆-Cycloalkyl steht oder für einen 5-6-gliedrigen Heterocyclus steht, der ein oder zwei Heteroatome, ausgewählt aus N-, O- und S-Atomen, enthält, wenn X N ist,
wobei Alkyl, Alkoxy, Aryl, Cycloalkyl, Heterocyclus und Heteroaryl optional substituiert sein können und die Substituenten einer oder mehrere, ausgewählt aus der Gruppe, bestehend aus Hydroxy, C₁-C₆-Alkylamino, Di(C₁-C₆-alkyl)amino, Carboxy, C₁-C₆-Alkyl, C₁-C₆-Alkoxy, Carboxy-C₁-C₆-alkyl und Oxo, sind.

2. Verfahren gemäß Anspruch 1, wobei die durch die obige Formel 1 dargestellte Verbindung durch Umsetzen der durch die obige Formel 3 dargestellten Verbindung in Gegenwart von mindestens einer Verbindung, ausgewählt aus der Gruppe, bestehend aus PBr₃, SOBr₂, HBr und LiBr, hergestellt wird.

3. Verfahren gemäß Anspruch 1, umfassend die Schritte: das Substituieren der durch die obige Formel 1 dargestellten Verbindung, um eine durch die nachstehende Formel 4 dargestellte Verbindung herzustellen;
das Reduzieren der durch die obige Formel 4 dargestellten Verbindung, um eine durch die Formel 5 dargestellte Verbindung herzustellen; und
das Umsetzen der durch die Formel 5 dargestellten Verbindung mit einem Keton oder Aldehyd, um eine durch die obige Formel 2 dargestellte Verbindung herzustellen: 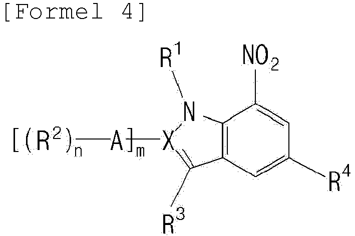 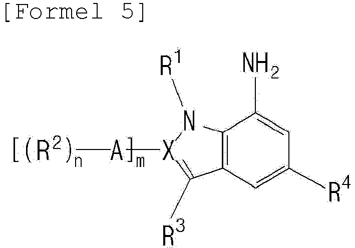
wobei in den obigen Formeln n, m, X, A, R1, R2, R3 und R4 die gleichen, wie in Anspruch 1 definiert, sind.

## Revendications

1. Procédé de préparation d'un composé représenté par la Formule 2 ci-dessous comprenant un composé représenté par la Formule 1 ci-dessous en tant qu'intermédiaire de réaction, dans lequel le composé représenté par la Formule 1 ci-dessous est préparé par bromation du composé représenté par la Formule 3 ci-dessous : 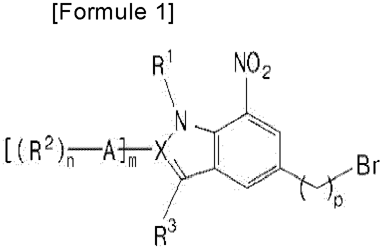 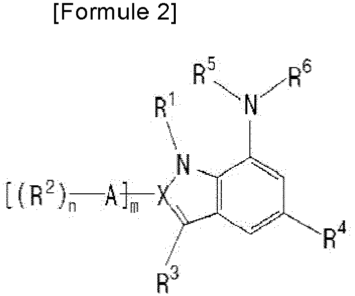 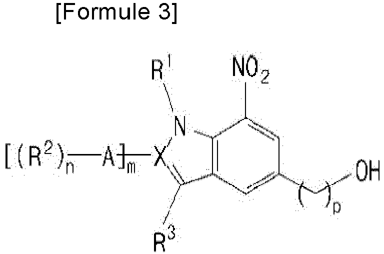
dans lequel, dans les formules ci-dessus,
n est un nombre entier de 1 à 3,
m est 0 ou 1,
p est un nombre entier de 1 à 3,
A représente un phényle, ou représente un hétéroaryle ou un hétérocycle à 5 chaînons dont chacun contient 1 à 3 hétéroatomes sélectionnés parmi les atomes N, O et S et est facultativement substitué par R, dans lequel R représente un hydrogène ou représente un alkyle en C₁-C₄ facultativement substitué par un hydroxy ou un amino,
X représente C ou N, à condition que m soit 0 lorsque X est N et que m soit 1 lorsque X est C,
R¹ représente un hydrogène, un alkyle en C₁-C₆ ou -(CH₂)ᵣNR⁸R⁹, dans lequel r est un nombre entier de 2 à 5, R⁸ et R⁹ représentent chacun indépendamment un hydrogène ou un alkyle en C₁-C₃, à condition que R¹ soit un hydrogène lorsque X est N,
R² représente un hydrogène, un halogène ou un alcoxy en C₁-C₆, ou représente -(CH₂)_{S}CO₂R⁸, -(CH₂)_{S}OR⁸, -(CH₂)ₛNR⁸R⁹, -NHR¹⁰,-N(H)S(O)₂R⁸ ou -NHC(O)R¹⁰, ou représente -(CH₂)ₛ-hétérocycle-R¹⁰ dans lequel le groupement hétérocycle est un noyau de 5 à 6 chaînons contenant un ou deux hétéroatomes sélectionnés parmi les atomes N, O et S, dans lequel s est un nombre entier de 0 à 3, R⁸ et R⁹ sont tels que définis ci-dessus, et R¹⁰ représente un hydrogène, un oxo, un alkylcarbonyle en C₁-C₆, un alcoxy en C₁-C₆ ou un alkyle en C₁-C₆, ou représente un hétérocycle de 5 à 6 chaînons contenant un ou deux atomes d'azote en tant qu'hétéroatome,
R³ représente un hydrogène, un halogène, un alkyle en C₁-C₆ ou un phényle, ou représente un -(CH₂)_{g}-hétérocycle dans lequel l'hétérocycle est un noyau de 5 à 6 chaînons contenant un ou deux hétéroatomes sélectionnés parmi les atomes N et O, dans lequel g est un nombre entier de 1 à 3, à condition que R³ soit un hydrogène ou un phényle lorsque X est N,
R⁴ représente -YR¹¹, dans lequel Y est une liaison directe ou représente -(CR⁸R⁹)ₑY'-, dans lequel e est un nombre entier de 0 à 3, et R⁸ et R⁹ sont tels que précédemment définis,
Y' est sélectionné parmi le groupe consistant en -O-, -C(O)- et -C(O)O-, R11 est sélectionné parmi le groupe consistant en un hydrogène, un halogène, un alkyle en C₁-C₆ et -(CH₂)ₜB-R¹³, t est un nombre entier de 0 à 3, B représente un hétérocycle de 5 à 6 chaînons contenant un ou deux hétéroatomes sélectionnés parmi les atomes N, O et S, ou représente un aryle en C₆-C₁₀, représente un hydrogène, un cyano, un halogène, un hydroxy, un oxo, un thiol, un carboxy ou un carboxy-alkyle en C₁-C₆, à condition que R⁴ soit un hydrogène ou un alkyle en C₁-C₆ lorsque X est N,
R⁵ représente un hydrogène, un alkyle en C₁-C₆, un cycloalkyle en C₃-C₆, un hétérocycle ou un hétérocyclyl-alkyle en C₁-C₆, dans lequel l'hétérocycle est un noyau de 3 à 8 chaînons contenant un à trois hétéroatomes sélectionnés parmi les atomes N et O, à condition que R⁵ soit un hydrogène lorsque X est N, et
R⁶ représente -(CR⁸R⁹)ᵤ-Z-D-W-R¹⁴, dans lequel u est un nombre entier de 0 à 3, Z représente une liaison directe ou est sélectionné parmi le groupe consistant en -C(O)- et -C(O)O-, D représente une liaison directe, un cycloalkyle en C₄-C₆, ou un hétéroaryle de 5 à 6 chaînons contenant un ou deux atomes N, ou représente un hétérocycle de 5 à 6 chaînons contenant un ou deux hétéroatomes sélectionnés parmi les atomes N, O et S, W représente une liaison directe, ou -NR⁸-, -C(O)-, -C(O)O-, -C(O)NR¹²- ou -S(O)_{y-}, R¹² représente un hydrogène, un alkyle en C₁-C₃ ou un aryle en C₆-C₁₀, y est un nombre entier de 1 ou 2, et R¹⁴ représente un hydrogène, un hydroxy, un alkyle en C₁-C₆, un hétérocycle de 5 à 6 chaînons contenant un à trois hétéroatomes sélectionnés parmi les atomes N, O et S, ou un alkyle en C₆-C₁₀-Ar-C₁-C₆, à condition que lorsque X est N, R⁶ représente un cycloalkyle en C₄-C₆, ou représente un hétérocycle de 5 à 6 chaînons contenant un ou deux hétéroatomes sélectionnés parmi les atomes N, O et S,
dans lequel l'alkyle, l'alcoxy, l'aryle, le cycloalkyle, l'hétérocycle et l'hétéroaryle peuvent être facultativement substitués, et les substituants sont un ou plusieurs composés sélectionnés parmi le groupe consistant en un hydroxy, un alkylamino en C₁-C₆, un di(alkyl en C₁-C₆)amino, un carboxy, un alkyle en C₁-C₆, un alcoxy en C₁-C₆, un carboxy-alkyle en C₁-C₆ et un oxo.

2. Procédé selon la revendication 1, dans lequel le composé représenté par la Formule 1 ci-dessus est préparé en faisant réagir le composé représenté par la Formule 3 ci-dessus en présence d'au moins un composé sélectionné parmi le groupe consistant en PBr₃, SOBr₂, HBr et LiBr.

3. Procédé selon la revendication 1, comprenant les étapes pour : substituer le composé représenté par la Formule 1 ci-dessus pour préparer un composé représenté par la Formule 4 ci-dessous ;
réduire le composé représenté par la Formule 4 ci-dessus pour préparer un composé représenté par la Formule 5 ; et
faire réagir le composé représenté par la Formule 5 avec une cétone ou un aldéhyde pour préparer un composé représenté par Formule 2 ci-dessus : 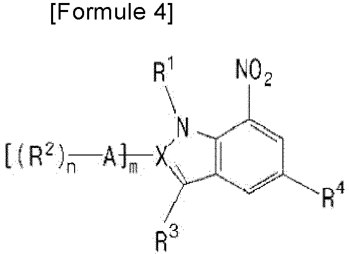 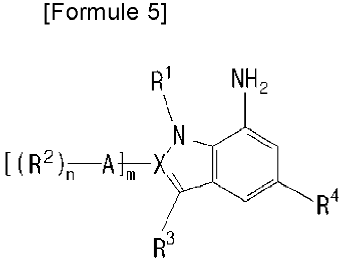 dans lequel, dans les Formules ci-dessus, n, m, X, A, R1, R2, R3 et R4 sont tels que définis dans la revendication 1.
